# EUROPEAN PATENT SPECIFICATION

(11) **EP 1 631 368 B1**
(45) Date of publication and mention of the grant of the patent: **11.05.2016**
(21) Application number: 03817122.9
(22) Date of filing: 06.06.2003
(51) Int. Cl.: B01D 39/08, B01D 46/10, B01D 46/42, A61L 9/00

(54) **MICROBICIDAL AIR FILTER**
MIKROBIOCIDER LUFTFILTER
FILTRE A AIR MICROBICIDE

(43) Date of publication of application: 08.03.2006
(73) Proprietor: PROTAIR-X HEALTH SOLUTIONS INC., Canada J4B 8P1 (CA)
(72) Inventor: BOLDUC, Normand, Québec H1G 5A2 (CA)
(74) Representative: Patentanwaltskanzlei Matschnig & Forsthuber OG
(86) International application number: PCT/CA2003/000858
(87) International publication number: WO 2004/108249

(56) References cited:
- WO-A-98/32494
- FR-A- 2 804 032
- US-A1- 2003 075 047
- US-B1- 6 540 807
- PATENT ABSTRACTS OF JAPAN vol. 1997, no. 07, 31 July 1997 (1997-07-31) & JP 09 084890 A (TOOA KK), 31 March 1997 (1997-03-31)
- PATENT ABSTRACTS OF JAPAN vol. 1998, no. 06, 30 April 1998 (1998-04-30) & JP 10 033701 A (TOPPAN PRINTING CO LTD), 10 February 1998 (1998-02-10)
- DATABASE WPI Section Ch, Week 200249 Derwent Publications Ltd., London, GB; Class A11, AN 2002-456777 XP002276463 & JP 2002 037706 A (HAYASHI M), 6 February 2002 (2002-02-06)
- PATENT ABSTRACTS OF JAPAN vol. 014, no. 284 (C-0730), 20 June 1990 (1990-06-20) & JP 02 088083 A (TAKAMITSU:KK;OTHERS: 03), 28 March 1990 (1990-03-28)
- DATABASE WPI Week 199019, Derwent Publications Ltd., London, GB; AN 1990-142944 & JP H0 288083 A (SASAKI KAGAKU KOGYO KK) 28 March 1990

## Description

### FIELD OF THE INVENTION

The present invention concerns air filters, more particularly microbicidal air filters and face masks according to the preamble of claim 1 and claim 12.

### BACKGROUND OF THE INVENTION

Removing airborne pathogens and environmental allergens is very important in environments that require high levels of air purity, such as in hospitals and in houses of people suffering from severe allergic responses to the aforesaid allergens. Typically, devices in the form of masks or in-air duct filters filter out particulate material during either air circulation or, in the case of facemasks, during inhalation and exhalation. The facemasks and air duct filters temporarily capture the pathogens and allergens, and particulate matter such as dust, on a surface of a filtering material. Once the filters reach a threshold limit or after a single use, they are typically discarded or in some cases, cleaned and reused. Many designs of filtering devices exist, examples of which are as follows:
- US Patent No. 1,319,763, issued October 28, 1919, to Drew for "Air filter for wall registers";
- US Patent No. 3,710,948, issued January 16, 1973, to Sexton for "Self-sustaining pocket type filter";
- US Patent No. 3,779,244, issued December 18, 1973, to Weeks for "Disposable face respirator";
- US Patent No. 3,802,429, issued April 9, 1974, to Bird for "Surgical face mask";
- US Patent No. 4,197,100, issued April 8,1980, to Hausheer for "Filtering member for filters";
- US Patent No. 4,798,676, issued January 17, 1989, to Matkovich for "Low pressure drop bacterial filter and method";
- US Patent No. 5,525,136, issued June 11, 1996, to Rosen for "Gasketed multi-media air cleaner';
- US Patent No. 5,747,053 issued May 5, 1998, to Nashimoto for "Antiviral filter air cleaner impregnated with tea extract"; and
- US Patent No. 5,906,677, issued May 25, 1999, to Dudley for "Electrostatic supercharger screen".

The aforesaid designs suffer from a number of important drawbacks. Disadvantageously, in the above-mentioned designs removal of the dirty filter or the facemask after use may cause non-immobilized pathogens or particulates to be dispersed into the air immediately around the user, which, if inhaled may be hazardous to the user. In addition, the designs may not immobilize the air borne pathogens and kill them in situ. Some of the designs incorporate viscous material into the filter material to capture particulate material. Some designs incorporate complex arrangements of filters inside cartridges, which may be impractical for use in air ducts or in facemasks. In some cases, fiberglass is used as part of the filter medium, which may be harmful to humans if located near the nose and mouth. In one design, disinfectant soaked cotton wool appears to be located in an air duct for aerosolizing into a room to maintain moisture content. Use of such a wet disinfectant may be harmful to humans in close proximity to the disinfectant and may not be appropriate for use in a facemask.

Also in documents US 2003/0075047 A1, JP 10-033701 A, JP 2002-37706 A and JP 02088083 A antibacterially equipped after-filter devices or facemasks are shown.

Further advantages of the invention will be in part obvious from an inspection of the accompanying drawings and a careful consideration of the following description.

### SUMMARY OF THE INVENTION

The present invention reduces the difficulties and disadvantages of the prior art by providing a microbicidal air filter according to claim 1, which captures and kills pathogenic microbes on a novel immobilization network of fibers. According to the invention, said microbicidal air filter is characterized by the features of the characterizing part of claim 1, namely the immobilization network includes a plurality of fibers, each of said fibers having substantially impregnated therein and integral thereto within the structure of the fibers an amount of at least one antimicrobial agent sufficient to substantially immobilize, retain and substantially inhibit the growth of microbes suspended in a volume of air moving through said air passageway, in which said substantially impregnated fibers are TRICLOSAN™ treated PVC organic fibers.

To achieve this, said fibers include that antimicrobial agent within their structure (impregnated therein), which substantially kills the microbes and retains them within the body of the fibers. This significantly reduces or essentially eliminates the problems associated with further release of the microbes from the filter after use and during disposal. Advantageously, the filter can be used as a facemask or in air-circulation ducts, typically as an after-filter or downstream of a filter, and can capture and kill a wide variety of microbes. The fibers are made of a material, which enables the filter to be washed and reused without significant loss of antimicrobial activity.

Accordingly, there is provided a microbicidal air filter for use with an air passageway, said air filter comprising: an immobilization network having substantially impregnated therein an amount of at least one antimicrobial substance sufficient to substantially immobilize, retain and kill microbes suspended in a volume of air moving through said air passageway, said immobilization network being substantially permeable to said air.

Accordingly, there is provided a microbicidal air filter for use with an air passageway, said air filter comprising: an immobilization network having substantially impregnated therein an amount of at least one antimicrobial substance sufficient to substantially immobilize, retain and substantially inhibit the growth of microbes suspended in a volume of air moving through said air passageway, said immobilization network being substantially permeable to said air.

The present invention reduces the difficulties and disadvantages of the prior art also by providing a microbicidal face mask according to claim 12.

According to the invention, said microbicidal facemask is characterized by the features of the characterizing part of claim 12, namely
- first and second air permeable screen elements secured together along respective peripheral edges, said screen elements defining a gap therebetween, said screen elements being configured and sized to fit over the mouth and nose of a user and to be secured thereto; and
- an air permeable immobilization network located in and substantially filling said gap; wherein
- the facemask in that said immobilization network includes a plurality of fibers, each of said fibers having substantially impregnated therein and integral thereto within the structure of the fibers an amount of at least one antimicrobial agent sufficient to substantially immobilize, retain and substantially inhibit the growth of microbes suspended in a volume of air moving through said network, in which said substantially impregnated fibers are TRICLOSAN™ treated PVC organic fibers.

Accordingly, there is provided a microbicidal face mask comprising: first and second air permeable screen elements secured together along respective peripheral edges, said screen elements defining a gap therebetween, said screen elements being configured and sized to fit over the mouth and nose of a user and to be secured thereto; an air permeable immobilization network located in and substantially filling said gap, said immobilization network having substantially impregnated therein an amount of at least one antimicrobial substance sufficient to substantially immobilize, retain and kill microbes suspended in a volume of air moving through said network.

Accordingly, there is provided a microbicidal face mask comprising: first and second air permeable screen elements secured together along respective peripheral edges, said screen elements defining a gap therebetween, said screen elements being configured and sized to fit over the mouth and nose of a user and to be secured thereto; an air permeable immobilization network located in and substantially filling said gap, said immobilization network having substantially impregnated therein an amount of at least one antimicrobial substance sufficient to substantially immobilize, retain and substantially inhibit the growth of microbes suspended in a volume of air moving through said network.

### BRIEF DESCRIPTION OF THE DRAWINGS

In the annexed drawings, like reference characters indicate like elements throughout.
**Figure 1** is a simplified exploded view of an embodiment of a filter;
**Figure 2** is a simplified partial cutaway view of a facemask with the filter;
**Figure 2a** is a simplified partial cutaway view of an alternative embodiment of a facemask;
**Figure 3** is a simplified exploded view of an embodiment of a filter in a frame;
**Figure 4** is a simplified exploded view of the filter with a primary filter;
**Figure 5** is a simplified exploded view of an air circulation system with a filter;
**Figure 6** is simplified front view of an alternative filter for use in the system of Figure 5;
**Figure 7** is a simplified front view of an alternative filter for use with the system of Figure 5, showing stitches as a fastening member;
**Figure 8** is a simplified front view of an alternative filter for use with the system of Figure 5, showing rivets as a fastening member; and
**Figure 9** is a cross sectional view taken along lines 9-9 of Figure 7.

### DETAILED DESCRIPTION OF THE INVENTION

With reference to the annexed drawings the preferred embodiments of the present invention will be herein described for indicative purposes and by no means as of limitation.

### Definitions

As used herein, the term "microbe" or "microbial" is intended to mean microorganisms including, but not limited to, bacteria, protozoa, viruses, molds and the like. Also included in this definition are dust mites.

As used herein, the term "antimicrobial agent" is intended to mean a compound that inhibits, prevents, or destroys the growth or proliferation of microbes such as bacteria, protozoa, viruses, molds and the like. Examples of antimicrobial agents as used herein include anti-bacterial agents, anti-viral agents, anti-mold agents, anti-yeast agents and anti-dust mite agents, or any combination thereof.

As used herein, the term "anti-bacterial agent" is intended to mean a compound that inhibits, prevents the growth of, or kills bacteria.

As used herein, the term "anti-viral agent" is intended to mean a compound that inhibits, prevents the growth of, or kills viruses.

As used herein, the term "anti-mold agent" is intended to mean a compound that inhibits, prevents the growth of, or kills molds.

As used herein, the term "anti-yeast agent" is intended to mean a compound that inhibits, prevents the growth of, or kills yeasts.

As used herein, the term "anti-dust mite agent" is intended to mean a compound that inhibits, prevents the growth of, or kills dust mites.

As used herein, the term "microbicidal" is intended to refer to the inhibition, growth prevention or killing properties of any of the aforesaid "agents", used either alone or in combination with each other.

### Preferred embodiments

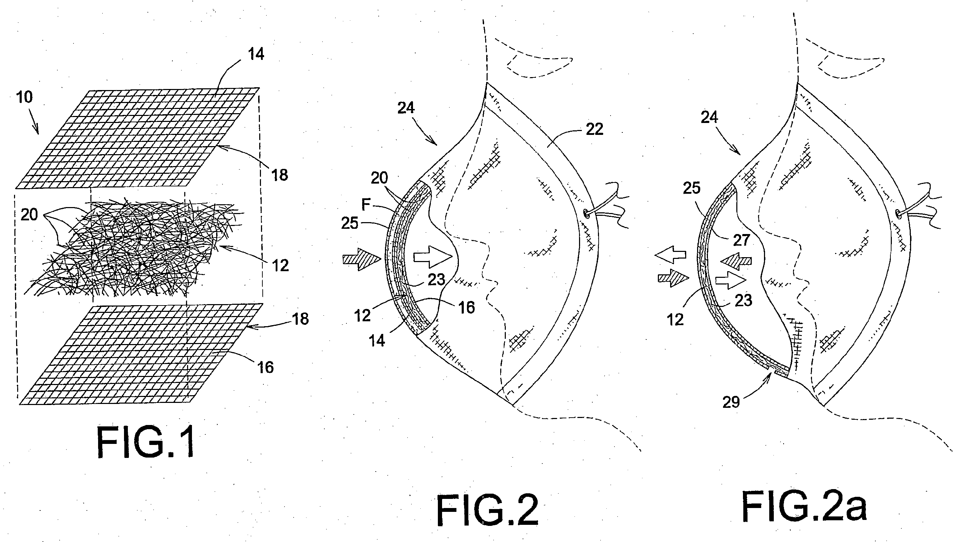

Referring now to Figure 1, a first embodiment of a microbicidal air filter shown generally at 10. Broadly speaking, the filter 10 includes an air permeable immobilization network 12, an air permeable first screen 14 and an air permeable second screen 16. The first screen 14 and the second screen 16 are merely acting to support the network 12 and to define a work area 18. One skilled in the art will recognize that the immobilization network 12 may be used independently of the screens 14 and 16.

The network 12 includes a mesh of fibers 20, which can be non-woven or woven depending on whether a soft or hard (rigid) network is desired. The network 12 may also include yarn such as cotton in which the fibers 20 are interwoven. Each fiber 20 includes a quantity of at least one antimicrobial agent that is fully impregnated and integral with the body of the fiber 20 thereby providing a large concentration of the antimicrobial agent over a large surface area. The fibers 20 are arranged such that they are permeable to air over the entire mesh, typically as a fine layer of so-called angels hair, of flaky mesh or the like.

According to the invention, the network is a fibrous material, namely TRICLOSAN™ treated PVC organic fiber.

TRICLOSAN™ is an antimicrobial agent, which reduces the growth or kills microbes such as bacteria, yeast and molds.

The fibrous material is either used pure (100%) or in blends, with a percentage of at least 30% volume, along with other types of fibers within woven or non-woven type fabrics, and which meet the requirements of an individual protective equipment (IPE). The fibrous material may also have other properties including, but not limited to, non-flammability, resistance to chemical products, ignition suppression, thermal insulation, and moisture management.

Typically, the fibrous material has porosity in the range of about 0.1 µm to about 3µm, although this depends upon the size of microbe to be retained.

Typically, the fibrous material has a density of between two grams per square foot (2 gr/ft²) to thirty grams per square foot (30 gr/ft²). More preferably, the density is around ten grams per square foot (10 gr/ft²).

As best illustrated in Figure 2, the filter 10 may be part of a facemask 24 of the type normally used by hospital workers and the like and which could be expandable (soft mask) or not (rigid mask), that are sometimes used in areas with pre-filtered air. The screens 14 and 16 are typically connected around a peripheral edge 22 and define a gap 23 therebetween. The network 12 can be attached to one of the aforesaid screens to provide both a physical barrier against particulate material and more importantly, to pathogenic microbes. The network 12 can be attached to the screens 14 or 16 using a VELCRO™ type fastener, stitches, bonding and the like, or inside an individual portable mask 24 that are worn in front of the nose/mouth area of the individual. A front mask screen 25 of the mask 24 acts as a primary filter located upstream of the network 12 to pre-filter the air by removing particulate material and microbes from the air passing therethrough along an air passageway, as shown by the arrows.

Alternatively, as best illustrated in Figure 2a, the network 12 may be located between the front screen 25 and a rear screen 27, such as commercially available filter masks, in the gap 23 of the facemask 24 to create a two-way system of filtration, as shown by the arrows. The front screen 25 may include a slit 29 to allow the network 12 to be inserted into the gap 23. This type of facemask 24 may be useful for people who are suffering from a respiratory infection and who still wish to work yet, don't wish to infect others by exhaling breath contaminated with pathogenic microbes.

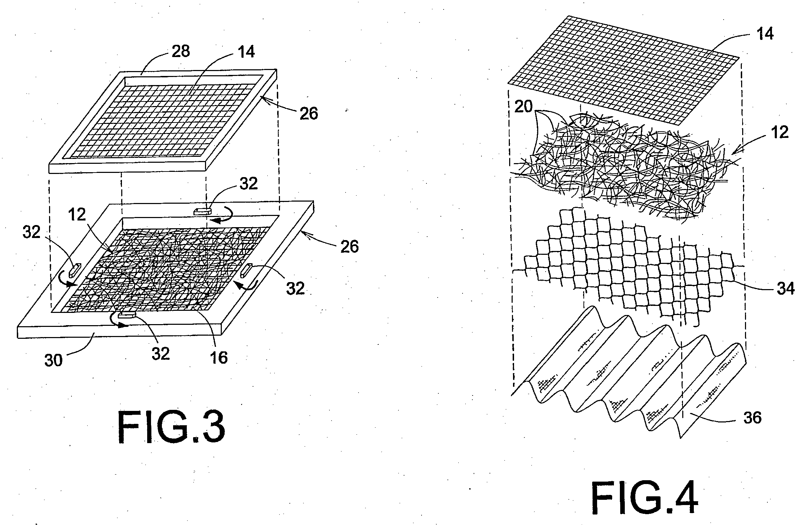

The screen elements 14, 16 can have different sizes and shapes and can be simple typical flexible or semi-flexible type screens as illustrated in Figure 1, made from aluminum, nylon, thermoplastic material, fiberglass type materials (usually not approved for mask applications), woven type fabrics or the like. As shown in Figure 3, the screen elements 14, 16 and the network 12 can be supported by a rigid frame 26, such as a standard aluminum screen frame, that is divided into two parts 28, 30 and integral with the screen elements 14, 16 respectively, to ensure rigidity and ease of installation. A fastening member 32 may be used to releasably connect the two screen elements 14, 16 together with the network 12 sandwiched therebetween and compressed to prevent it from being displaced by the air flowing therethrough. The fastening member 32 may be a pivoting retainer pivoting on one of the parts 28, 30 to retain the other part against the same. Alternatively, as best illustrated in Figure 4, a rigid screen 34 of any existing air filter 36 may also be used.

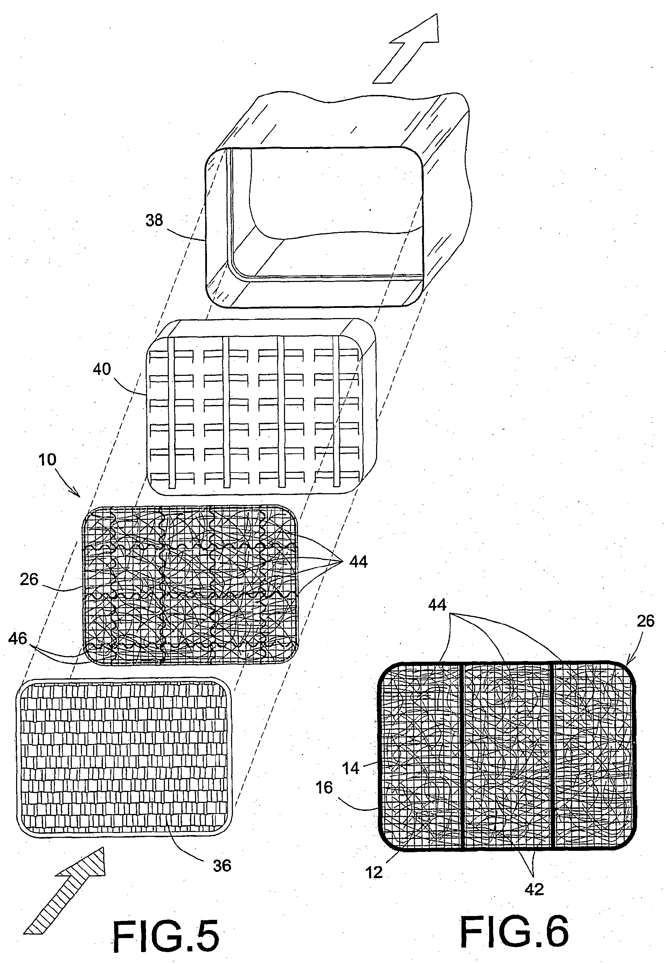

Referring now to Figures 5 and 6, the filter 10 is illustrated installed inside an air duct 38 downstream of the air filter 36 and upstream of an air heating system 40 (the arrows in Figure 5 show the air passageway) such that the air passing through the network 12 is pre-filtered. The frame 26 generally encloses the screen elements 14, 16 but also includes intermediate reinforcing rods 42 used to subdivide the screen elements 14, 16 into a plurality of smaller sub-elements 44 to constrain the network 12 to remain in place between the two elements 14, 16. Alternatively, as best seen in Figure 6, the frame 26 is a thin metallic rod onto which the screens 14, 16 are attached, with reinforcing rods 42 providing additional support to the screen elements 14, 16 and to the network 12 and to provide the aforesaid sub-elements 44.

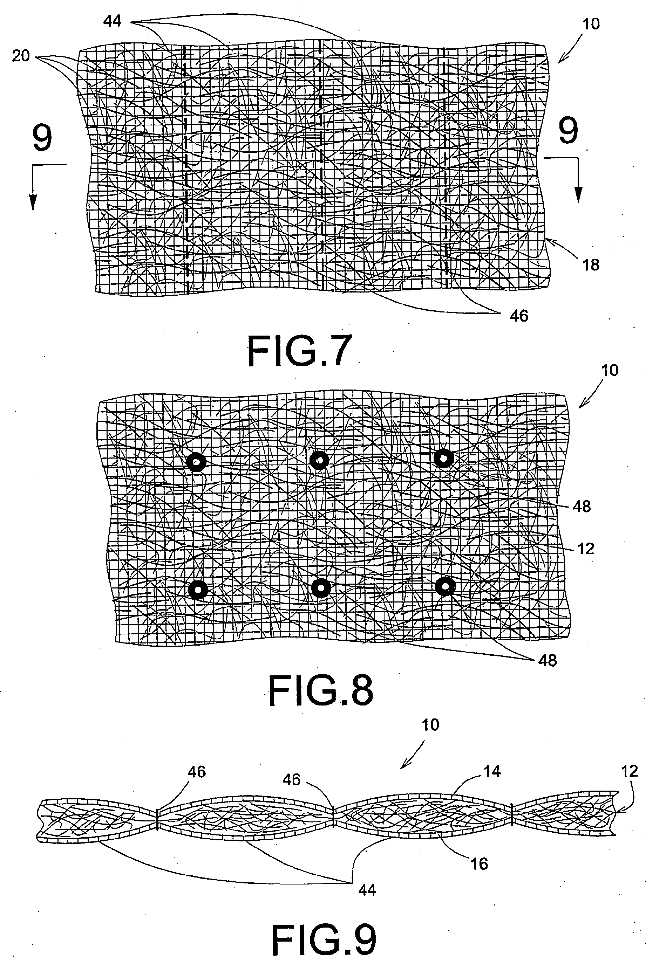

Referring now to Figures 5, 7, 8 and 9, other types of fastening members 32 are illustrated. One preferable type of fastening member 32 includes a plurality of stitches 46 which may be arranged in a variety of patterns, for example wavy lines or straight lines. The stitches 46 pass through the network 12 and divide the network into subdivisions 44, as previously described. Alternatively, as best illustrated in Figure 8, the fastening members 32 may also include rivets 48, which pass through the network 12.

### EXAMPLES

The present invention is illustrated in further detail by the following non-limiting examples.

### Example 1

### Evaluation of microbicidal and filtering capacity of rigid and soft facemasks

As shown in Table 1, two facemasks of the present invention were compared to a commercially available facemask^{1,2,3} for their antimicrobial and retaining capabilities against a panel of bacteria and molds of various sizes^{4,5,6,7}. The NB rigid and soft masks used in Examples 1 and 2 were both equipped with a network 12 of PVC organic fiber containing TRICLOSAN™. The NB soft mask was composed of a double covering of woven type fabric containing 76% w/w THERMOVYL-ZCB™ fibers and 24% w/w polyester (although any other woven type fabric such as cotton or the like could have been used) stitched to each other at their periphery, within which the network 12 was located (see Figure 2a above). The NB rigid mask was made of two conventional commercially available anti-dust masks, which were inserted one inside the other, between which the network of PVC organic fiber containing TRICLOSAN™ was located.

An air contamination chamber^{5,8,9} was used to measure the filtering capacity of a mask containing the network. The chamber includes a perforated bottle containing a predetermined quantity of lyophilized microorganisms. The chamber is installed on a microbiologic air-sampler. The test mask was installed at the interface between the contaminated air chamber and the air sampler. A negative pressure was generated in the air chamber, which caused the lyophilized microorganisms to move towards the mask. A culturing medium was located downstream of the mask to detect any breakthrough of the mask.

**TABLE 1**

| **Microorganisms** | **Size (µm)** | **Filtration efficiency (%)** | | |
|---|---|---|---|---|
| | | **NBRM** | **NBSM** | **3M*** |
| **Bacteria** | | | | |
| Mycobacteria tuberculosis | 0.2-0.7 x 1.0-10 | 100 | 100 | 95 |
| Proteus spp. | 0.4-0.8 x 1-3 | 100 | 100 | |
| Pseudomonas aureginosa | 0.5-1.0 x 1.5-5 | 100 | 100 | |
| Staphylococcus aureus | 0.5 x 1.5 | 100 | 100 | |
| Streptococcus pneumoniae | 0.5-1.5 | 100 | 100 | |
| Haemophilius influenze | 1 | 100 | 100 | |
| Anthrax | 1-1.5 x 3-5 | 100 | 100 | |

| **Moulds** | | | | |
|---|---|---|---|---|
| Acremonium strictum | 3.3-5.5 (7) x 0.9 x 1.8 | 100 | 100 | 96 |
| Aspergillus versicolor | 2-3.5 | 100 | 100 | |
| Penicillium griseofulvum | 2.5-3.5 x 2.2-2.5 | 100 | 100 | |
| Neosartorya fischeri | 2x2.5 | 100 | 100 | |

| | | | | |
|---|---|---|---|---|
| NBRM=Rigid mask NBSM=Soft mask * Data from technical specification² | | | | |

### Example 2

### Evaluation of filtering of small particles

The filtering capacity of the three masks of Example 1 was tested against two particulate materials of 0.3 µm particle size using essentially the same apparatus as in Example 1. A cartridge capturing membrane located downstream from an air pump, in this case, captured breakthrough particulates. The air pump creates a negative pressure downstream of the mask. The two particulate materials chosen were sodium chloride and dioctyl phthalate.

**TABLE 2**

| **Particulate material** | **Size (µm)** | **Filtration efficiency (%)** | | |
|---|---|---|---|---|
| | | **NBRM** | **NBSM** | **3M*** |
| Sodium chloride (NaCl) | 0.3 | 100 | 100 | 95 |
| Dioctylphthalate (DOP) | 0.3 | 100 | 100 | |

| | | | | |
|---|---|---|---|---|
| NBRM=Rigid mask NBSM=Soft mask * Data from technical specification² | | | | |

### Example 3 (not according to the invention)

### Evaluation of microbicidal and filtering capacity of a ventilation system filter

The antimicrobial capacity of a filter of the embodiment of Figure 3 with RHOVYL'A.S.+™ fibers was evaluated after 0, 7, 14, and 21 days installation in a ventilation system in a house. The results are illustrated in Tables 3 to 6 below.

The filters were removed after the aforesaid times and analysed using the Samson method¹⁰. The fibrous material (1 g) of each filter was diluted with demineralised, sterilized water (9 mL) and then serially diluted.

The calculation of total amount of bacteria, yeast and molds were done using hemacytometry. The calculation of the total amount of viable bacteria, yeasts and molds were determined following a culture of the serial dilutions on appropriate media. The aerobic viable bacteria were cultured on soya agar-agar (TSA, Quelab), whereas the yeasts and molds were cultured on HEA supplemented with gentamycin (0.005% p/v) and oxytetracycline (0.01% p/v) to limit bacterial growth. HEA's pH of 4.8 +/- 0.2 allows the germination of spores and development of mycelens. After the incubation period, the calculation of microbial colonies was carried out using a colony meter (Accu-Lite™, Fisher). The morphotype of the bacterial colonies was identified by Gram staining (see Table 5).

Concerning the yeasts and molds calculation, each macroscopically distinct mold colony was identified by gender and/or species using microscopy.

Mold slides were prepared using the adhesive tape method¹¹. This technique maintains the integrity of the mold structures by fixing them on the sticky side of the tape. Once collected, the molds were stained with lactophenol and observed at a magnification of 10x and 40x. Using identification keys^{12,13,14,15}, the molds were identified. In this experiment only colonies that produced spores were identified.

**TABLE 3: Bacterial filtering**

| **After filter** | **Calculated bacteria (UFC/g)** | | |
|---|---|---|---|
| Time (days) | Viable | Non-viable | Total |
| 0 | 6000 (3.43%) | 169000 (96.57%) | 175000 (100%) |
| 7 | 9000 (2.75%) | 318000 (97.25%) | 327000 (100%) |
| 14 | 27000 (2.21%) | 1193000 (97.79%) | 1220000 (100%) |
| 21 | 70000 (1.88%) | 3650000 (98.12%) | 3720000 (100%) |

**TABLE 4: Fungal filtering**

| **After filter** | **Calculated fungi (UFC/g)** | | |
|---|---|---|---|
| Time (days) | Viable | Non-viable | Total |
| 0 | 29000 (11.74%) | 218000 (88.26%) | 247000 (100%) |
| 7 | 110000 (10.19%) | 970000 (89.81%) | 1080000 (100%) |
| 14 | 230000 (8.75%) | 2400000 (91.25%) | 2630000 (100%) |
| 21 | 1640000 (7.24%) | 21000000 (92.76%) | 22640000 (100%) |

**TABLE 5: Identification of bacterial morphotypes**

| **After filter (days)** | **Bacterial morphotypes** |
|---|---|
| 0 | 78.4% Cocci Gram positive |
| | 21.6% Rod Gram negative |
| 7 | 84.3% Cocci Gram positive |
| | 15.7% Rod Gram negative |
| 14 | 86.7% Cocci Gram positive |
| | 13.3% Rod Gram negative |
| 21 | 88.9% Cocci Gram positive |
| | 11.1 % Rod Gram negative |

**TABLE 6: Identification of mold species**

| **After filter (days)** | **Mold species** |
|---|---|
| 0 | *Aspergillus niger, Cladosporium cladosporioides, Cladosporium herbarum, Penicillium sp., yeasts* |
| 7 | *Aspergillus niger, Cladosporium cladosporioides, Cladosporium herbarum, Penicillium sp., yeasts* |
| 14 | *Alternaria alternata, Arthrinium sp., Aspergillus niger, Cladosporium sp., Geotrichum sp., Penicillium sp., yeasts* |
| 21 | Aspergillus niger, *Cladosporium cladosporioides, Cladosporium herbarum, Penicillium sp., yeasts* |

### Discussion

To date, commercially available masks have been hampered by their inability to capture and kill in excess of 95% of microorganisms. A study of a microbicidal network of the present invention, in the form of the facemasks and filters in a ventilation system, has demonstrated a significant improvement in capturing and killing efficiency (Tables 1 to 6).

Tables 1 and 2 illustrated the effectiveness of PVC organic fiber containing TRICLOSAN™ as particulate filters, anti-bacterial and anti-mold filters. For both the soft facemask and the rigid facemask, the anti-microbial and particulate filtering capacities were 100% compared to the corresponding capacities for a commercially available mask (95 to 96%).

If desired, the filter can be cleaned and reused without a significant loss of the aforesaid capacities (results not shown).

A key feature of the filter 10, whether it be in the aforesaid facemasks or the circulation system duct filter, is its ability to immobilize, retain and kill or inhibit the growth of a wide variety of microbes, which come into contact with the network 12 of fibers 20. Air that is either pre-filtered, in the case of the circulation system, or inhaled/exhaled through the facemask by the user, often includes residual microbes that have either passed through the primary filter or the filter has failed to immobilize them. In the case where a person who uses the facemask of the present invention and who has an upper respiratory infection, such as influenza, tuberculosis, anthrax, severe acute respiratory syndrome (SARS) and the like, can significantly reduce or essentially eliminate further infection to other people. Similarly, air that is contaminated with pathogenic microbes can be filtered before entering into the nose and mouth area of the user. The flow of air is shown by the arrows in Figures 2, 2a, and 5, in which air contaminated with microbes is shown as hatched lines and non-hatched arrows show clean, filtered air.

### References (incorporated herein by reference)

1. National Institute for Occupational Safety and Health. NIOSH respirator decision logic. Cincinnati, Ohio: Department of Health and Human Services, Public Health service, CDC, 1987:13-9; DHHS publication no. (NIOSH) 87-108.
2. TB Respiratory Protection Program In Health Care Facilities Administrator's Guide, (http://www.cdc.gov/niosh/99-143.html).
3. 3M Soins de santé Canada; Une protection fiable a chaque respiration; 3M® 2002.
4. MMWR; Laboratory Performance Evaluation of N95 Filtering Facepiece Respirators, 1996 (December 11, 1998).
5. Edwin H.Lennette, Albert Balows, William J.Hausler, Jr.H.Jean Shadomy, 1985, Manual of Clinical Microbiology.
6. Robert A.Samson, Ellen S. van Reenen-Hoekstra, 1990, Introduction to food-borne Fungi.
7. G. Nolt, Noel R. Krieg, Peter H. A. Sneath, James T. Staley, Stanley, T. Williams, 1994, Bergey's Manual of Determinative bacteriology.
8. Fradkin A (1987) Sampling of microbiological contaminants in indoor air, In: sampling and calibration for atmospheric measurements ASTM Special Technical Publication, 957:66-77.
9. 42 CFR Part 84 Respiratory Protective Devices, (http://www.cdc.gov/niosh/pt84abs2.html).
10. Samson, RA. 1985. Air sampling methods for biological contaminants. Document de travail fourni au Groupe sur les champignons dans l'air des maisons de Santé et Bien-être social Canada, Ottawa, Ontario, K1A 1L2.
11. Koneman, W.E. et G.D. Roberts. 1985. Practical laboratory mycology. 3rd ed. Williams and Wilkins. Baltimore. MD.
12. Domsch, K.H., W. Gams et T.-H. Anderson. 1980. Compendium of soil fungi. Academic Press. London.
13. Larone, D.H. 1987. Medically important fungi. A guide to identification. New York. Elsevier Science Publishing Co. Inc.
14. Malloch, D. 1981. Moulds, their isolation, cultivation and identification. Toronto: University of Toronto Press. 97 p.
15. St-Germain, G. et R.C. Summerbell. 1996. Champignons filamenteux d'intérêt médical : Caractéristiques et identification. Star Publishing Company. Belmont. CA.

## Claims

1. Microbicidal air filter (10) for use with an air passageway and having an immobilization network (12) being substantially permeable to said air, said air filter (10) being **characterized in that:**
- the immobilization network (12) includes a plurality of fibers (20), each of said fibers having substantially impregnated therein and integral thereto within the structure of the fibers (20) an amount of at least one antimicrobial agent sufficient to substantially immobilize, retain and substantially inhibit the growth of microbes suspended in a volume of air moving through said air passageway, in which said substantially impregnated fibers (20) are TRICLOSAN™ treated PVC organic fibers.

2. The filter (10), according to claim 1, in which said plurality of fibers (20) are arranged in a mesh, said mesh defining a plurality of air spaces between said fibers (20).

3. The filter (10), according to claim 1, in which said antimicrobial agent kills microbes suspended in the volume of air.

4. The filter (10), according to claim 2, in which said substantially impregnated fibers (20) are tightly woven or loosely woven.

5. The filter (10), according to claim 1, in which said immobilization network (12) is an after-filter so that the air is pre-filtered prior reaching the air passageway.

6. The filter (10), according to claim 1, in which said air fitter (10) is a facemask (24) configured and sized to fit over the nose and mouth of a user and to be secured therearound.

7. The filter (10), according to claim 1, in which said air filter (10) is an air duct filter configured and sized to fit in an air duct system (40) forming the air passageway.

8. The filter (10), according to claim 7, in which said air filter (10) further includes:
- first and second air permeable screen elements (14,16) securable together along respective peripheral edges (22), said screen elements (14,16) being configured and sized to fit in the air duct system (38) and to be secured therein;
- said air permeable immobilization network (12) being located substantially between said first and second screen elements (14,16).

9. The filter (10), according to claim 8, in which a fastening member (32) connects said first and second air permeable screen elements (14,16) together to sandwich said immobilization network (12) therebetween.

10. The filter (10), according to claim 9, in which said fastening member (32) includes a frame (26) for connecting said first and second screen elements (14,16) together.

11. The filter (10), according to claim 10, in which said fastening member (32) further includes a plurality of stitches (46) located through said immobilization network (12) to divide said immobilization network (12) into subdivisions (44).

12. Microbicidal facemask (24) having:
- first and second air permeable screen elements (14,16) secured together along respective peripheral edges (22), said screen elements (14,16) defining a gap (23) therebetween, said screen elements (14,16) being configured and sized to fit over the mouth and nose of a user and to be secured thereto; and
- an air permeable immobilization network (12) located in and substantially filling said gap (23);
- the facemask (24) being **characterized in that** said immobilization network (12) includes a plurality of fibers (20), each of said fibers having substantially impregnated therein and integral thereto within the structure of the fibers (20) an amount of at least one antimicrobial agent sufficient to substantially immobilize, retain and substantially inhibit the growth of microbes suspended in a volume of air moving through said network (12),
in which said substantially impregnated fibers (20) are TRICLOSAN™ treated PVC organic fibers.

13. The facemask (24), according to claim 12, in which said a plurality of fibers (20) are arranged in a mesh, said mesh defining a plurality of air spaces between said fibers (20).

14. The facemask (24), according to claim 12, in which said antimicrobial agent kills microbes suspended in the volume of air.

15. The facemask (24), according to claim 13, in which said substantially impregnated fibers (20) are tightly woven or loosely woven.

16. The facemask (24), according to claim 12, in which said immobilization network (12) is an after-filter so that the air is pre-filtered prior reaching the air passageway.

17. The facemask (24), according to claim 12, in which said first air permeable screen element (14) includes a slit (29) located therein of sufficient size to allow said immobilization network (12) to be positioned in said gap (23).

18. The filter (10), according to claim 1, in which the at least one antimicrobial agent is impregnated in a way within the structure of the fibers (20) that enables the filter (10) to be washed and reused significantly loss-free of antimicrobial activity of the at least one antimicrobial agent.

## Patentansprüche

1. Mikrobizider Luftfilter (10) zur Verwendung mit einem Durchlassweg für Luft, aufweisend ein Immobilisierungsnetzgebilde (12), das für die Luft im Wesentlichen durchgängig ist,
**dadurch gekennzeichnet, dass**
das Immobilisierungsnetzgebilde (12) eine Vielzahl von Fasern (20) beinhaltet, von denen jede, im Wesentlichen hinein imprägniert und als Bestandteil in der Struktur der Fasern (20), eine Menge zumindest eines antimikrobiellen Wirkstoffs hat, die dafür ausreicht, Mikroben, die in einem sich durch den Luft-Durchlassweg bewegenden Luft-Volumen suspendiert sind, zu immobilisieren, festzuhalten und deren Wachstum im Wesentlichen zu hemmen,
worin die im Wesentlichen imprägnierten Fasern (20) mit TRICLOSAN™ behandelte organische PVC-Fasern sind.

2. Luftfilter (10) nach Anspruch 1, bei welchem die Vielzahl von Fasern (20) in einem Maschenwerk angeordnet sind, welches eine Vielzahl von Lufträumen zwischen den Fasern (20) definiert.

3. Luftfilter (10) nach Anspruch 1, bei welchem der antimikrobielle Wirkstoff Mikroben, die in dem Volumen der Luft suspendiert sind, abtötet.

4. Luftfilter (10) nach Anspruch 2, bei welchem die im Wesentlichen imprägnierten Fasern (20) eng gewebt oder locker gewebt sind.

5. Luftfilter (10) nach Anspruch 1, bei welchem das Immobilisierungsnetzgebilde (12) ein Nachfilter ist, sodass die Luft vor dem Erreichen des Luft-Durchlassweges vorgefiltert wird.

6. Luftfilter (10) nach Anspruch 1, bei welchem der Luftfilter (10) eine Gesichtsmaske (24) ist, die zum Aufsetzen über Nase und Mund eines Benutzers und Befestigen darum ausgelegt und größenmäßig angepasst ist.

7. Luftfilter (10) nach Anspruch 1, wobei der Luftfilter (10) ein Filter einer Luftleitung ist, der zum Einfügen in ein Lüftungssystem (40), das den Luft-Durchlassweg bildet, ausgelegt und größenmäßig angepasst ist.

8. Luftfilter (10) nach Anspruch 7, wobei der Luftfilter (10) ferner aufweist:
- erste und zweite luftdurchlässige Siebelemente (14, 16), die aneinander jeweils entlang Randkanten (22) befestigbar sind und die zum Einfügen in das Lüftungssystem (38) und zum Befestigen darin ausgelegt und größenmäßig angepasst sind;
- das luftdurchlässige Immobilisierungsnetzgebilde (12), das im Wesentlichen zwischen dem ersten und dem zweiten Siebelement (14, 16) angeordnet ist.

9. Luftfilter (10) nach Anspruch 8, bei welchem ein Befestigungsteil (32) das erste und das zweite luftdurchlässige Siebelement (14, 16) miteinander zum Ausbilden einer Sandwich-Anordnung mit dem Immobilisierungsnetzgebilde (12) dazwischen verbindet.

10. Luftfilter (10) nach Anspruch 9, bei welchem das Befestigungsteil (32) einen Rahmen (26) zum gegenseitigen Verbinden des ersten und zweiten Siebelements (14, 16) beinhaltet.

11. Luftfilter (10) nach Anspruch 10, bei welchem das Befestigungsteil (32) ferner eine Vielzahl von durch das Immobilisierungsnetzgebilde (12) eingebrachte Nähte (46) zum Aufteilen des Immobilisierungsnetzgebildes (12) in Unterabteilungen (44) beinhaltet.

12. Mikrobizide Gesichtsmaske (24) mit:
- einem ersten und einem zweiten luftdurchlässigen Siebelement (14, 16), die aneinander jeweils entlang Randkanten (22) befestigt sind, wobei zwischen den Siebelementen (14, 16) ein Spalt (23) definiert ist, wobei die Siebelemente (14, 16) zum Aufsetzen über Mund und Nase eines Benutzers und Befestigen daran ausgelegt und größenmäßig angepasst sind; und
- einem luftdurchlässigen Immobilisierungsnetzgebilde (12), das in dem Spalt (23) angeordnet ist und diesen im Wesentlichen ausfüllt,
**dadurch gekennzeichnet, dass**
das Immobilisierungsnetzgebilde (12) eine Vielzahl von Fasern (20) beinhaltet, von denen jede, im Wesentlichen hinein imprägniert und als Bestandteil in der Struktur der Fasern (20), eine Menge zumindest eines antimikrobiellen Wirkstoffs hat, die dafür ausreicht, Mikroben, die in einem sich durch den Luft-Durchlassweg bewegenden Luft-Volumen suspendiert sind, zu immobilisieren, festzuhalten und deren Wachstum im Wesentlichen zu hemmen,
worin die im Wesentlichen imprägnierten Fasern (20) mit TRICLOSAN™ behandelte organische PVC-Fasern sind.

13. Gesichtsmaske (24) nach Anspruch 12, bei welcher die Vielzahl von Fasern (20) in einem Maschenwerk angeordnet sind, welches eine Vielzahl von Lufträumen zwischen den Fasern (20) definiert.

14. Gesichtsmaske (24) nach Anspruch 12, bei welcher der antimikrobielle Wirkstoff Mikroben, die in dem Volumen der Luft suspendiert sind, abtötet.

15. Gesichtsmaske (24) nach Anspruch 13, bei welcher die im Wesentlichen imprägnierten Fasern (20) eng gewebt oder locker gewebt sind.

16. Gesichtsmaske (24) nach Anspruch 12, bei welcher das Immobilisierungsnetzgebilde (12) ein Nachfilter ist, sodass die Luft vor dem Erreichen des Luft-Durchlassweges vorgefiltert wird.

17. Gesichtsmaske (24) nach Anspruch 12, bei welcher das erste luftdurchlässige Siebelement (14) einen darin befindlichen Schlitz (29) mit ausreichender Größe zum Ermöglichen des Platzierens des Immobilisierungsnetzgebildes (12) in dem Spalt (23) aufweist.

18. Luftfilter (10) nach Anspruch 1, bei welchem der antimikrobielle Wirkstoff in einer Weise in der Struktur der Fasern (20) imprägniert ist, die Waschen und Wiederverwenden des Filters (10) im Wesentlichen frei von Verlust der antimikrobiellen Wirkung des zumindest einen antimikrobiellen Wirkstoffs gestattet.

## Revendications

1. Filtre à air microbicide (10) pour une utilisation avec un passage d'air et ayant un réseau d'immobilisation (12) sensiblement perméable audit air, ledit filtre à air (10) étant **caractérisé par le fait que :**
- le réseau d'immobilisation (12) comprend une pluralité de fibres (20), chacune desdites fibres ayant, sensiblement imprégnée à l'intérieur de celle-ci et solidaire de celle-ci à l'intérieur de la structure des fibres (20), une quantité d'au moins un agent antimicrobien suffisante pour sensiblement immobiliser, retenir et sensiblement inhiber la croissance de microbes en suspension dans un volume d'air se déplaçant à travers ledit passage d'air, dans lequel lesdites fibres sensiblement imprégnées (20) sont des fibres organiques de PVC traitées par TRICLOSAN™.

2. Filtre (10) selon la revendication 1, dans lequel ladite pluralité de fibres (20) sont disposées en un treillis, ledit treillis définissant une pluralité d'espaces d'air entre lesdites fibres (20).

3. Filtre (10) selon la revendication 1, dans lequel ledit agent antimicrobien tue des microbes en suspension dans le volume d'air.

4. Filtre (10) selon la revendication 2, dans lequel lesdites fibres sensiblement imprégnées (20) sont tissées de façon serrée ou tissées de façon lâche.

5. Filtre (10) selon la revendication 1, dans lequel ledit réseau d'immobilisation (12) est un filtre finisseur, de telle sorte que l'air est préfiltré avant d'atteindre le passage d'air.

6. Filtre (10) selon la revendication 1, dans lequel ledit filtre à air (10) est un masque facial (24) configuré et dimensionné pour s'adapter sur le nez et la bouche d'un utilisateur et être fixé autour de ceux-ci.

7. Filtre (10) selon la revendication 1, dans lequel ledit filtre à air (10) est un filtre de conduit d'air configuré et dimensionné pour s'adapter dans un système de conduit d'air (40) formant le passage d'air.

8. Filtre (10) selon la revendication 7, dans lequel ledit filtre à air (10) comprend en outre :
- des premier et second éléments écrans perméables à l'air (14, 16) aptes à être fixés ensemble le long de bords périphériques respectifs (22), lesdits éléments écrans (14, 16) étant configurés et dimensionnés pour s'adapter dans le système de conduit d'air (38) et être fixés à l'intérieur de celui-ci ;
- ledit réseau d'immobilisation perméable à l'air (12) étant situé sensiblement entre lesdits premier et second éléments écrans (14, 16).

9. Filtre (10) selon la revendication 8, dans lequel un élément de fixation (32) relie lesdits premier et second éléments écrans perméables à l'air (14, 16) ensemble pour prendre en sandwich ledit réseau d'immobilisation (12) entre eux.

10. Filtre (10) selon la revendication 9, dans lequel ledit élément de fixation (32) comprend un cadre (26) pour relier lesdits premier et second éléments écrans (14, 16) ensemble.

11. Filtre (10) selon la revendication 10, dans lequel ledit élément de fixation (32) comprend en outre une pluralité de mailles (46) situées sur tout ledit réseau d'immobilisation (12) pour diviser ledit réseau d'immobilisation (12) en sous-divisions (44).

12. Masque facial microbicide (24) ayant :
- des premier et second éléments écrans perméables à l'air (14, 16) fixés ensemble le long de bords périphériques respectifs (22), lesdits éléments écrans (14, 16) définissant un intervalle (23) entre eux, lesdits éléments écrans (14, 16) étant configurés et dimensionnés pour s'adapter sur la bouche et le nez d'un utilisateur et être fixés à ceux-ci ; et
- un réseau d'immobilisation perméable à l'air (12) situé dans et remplissant sensiblement ledit intervalle (23) ;
le masque facial (24) étant **caractérisé par le fait que** ledit réseau d'immobilisation (12) comprend une pluralité de fibres (20), chacune desdites fibres ayant, sensiblement imprégnée à l'intérieur de celle-ci et solidaire de celle-ci dans la structure des fibres (20), une quantité d'au moins un agent antimicrobien suffisante pour sensiblement immobiliser, retenir et sensiblement inhiber la croissance de microbes en suspension dans un volume d'air se déplaçant à travers ledit réseau (12),
dans lequel lesdites fibres sensiblement imprégnées (20) sont des fibres organiques de PVC traitées par TRICLOSAN™.

13. Masque facial (24) selon la revendication 12, dans lequel ladite pluralité de fibres (20) sont disposées en un treillis, ledit treillis définissant une pluralité d'espaces d'air entre lesdites fibres (20).

14. Masque facial (24) selon la revendication 12, dans lequel ledit agent antimicrobien tue des microbes en suspension dans le volume d'air.

15. Masque facial (24) selon la revendication 13, dans lequel lesdites fibres sensiblement imprégnées (20) sont tissées de façon serrée ou tissées de façon lâche.

16. Masque facial (24) selon la revendication 12, dans lequel ledit réseau d'immobilisation (12) est un filtre finisseur, de telle sorte que l'air est préfiltré avant d'atteindre le passage d'air.

17. Masque facial (24) selon la revendication 12, dans lequel ledit premier élément écran perméable à l'air (14) comprend une fente (29) située à l'intérieur de celui-ci, de dimension suffisante pour permettre audit réseau d'immobilisation (12) d'être positionné dans ledit intervalle (23).

18. Filtre (10) selon la revendication 1, dans lequel l'au moins un agent antimicrobien est imprégné à l'intérieur de la structure des fibres (20) d'une manière qui permet au filtre (10) d'être lavé et réutilisé sensiblement sans perte d'activité antimicrobienne de l'au moins un agent antimicrobien.
